# EUROPEAN PATENT SPECIFICATION

(11) **EP 3 471 791 B1**
(45) Date of publication and mention of the grant of the patent: **05.08.2020**
(21) Application number: 16736622.8
(22) Date of filing: 15.06.2016
(51) Int. Cl.: A61L 31/04, A61L 31/10, A61L 31/12, A61L 31/14

(54) **MULTIFUNCTIONAL HERNIA PATCH**
MULTIFUNKTIONALES HERNIENPFLASTER
TIMBRE MULTIFONCTIONNEL POUR HERNIE

(43) Date of publication of application: 24.04.2019
(73) Proprietor: Tubitak, 06100 Ankara (TR)
(72) Inventor: SEZER, Serdar, 41470 Kocaeli (TR); AYDEMIR SEZER, Ümran, 41470 Kocaeli (TR); DOGAN, Hacer, 41470 Kocaeli (TR); AKTEKIN, Ali, 41470 Kocaeli (TR); SANKO, Vildan, 41470 Kocaeli (TR); HAYDANLI, Selçuk, 41470 Kocaeli (TR); AKER, Fugen, 41470 Kocaeli (TR)
(86) International application number: PCT/IB2016/053534
(87) International publication number: WO 2017/216609

(56) References cited:
- WO-A2-2008/075398
- WO-A2-2009/010879
- AU-B2- 2012 201 639
- US-A1- 2007 198 040
- DATABASE WPI Week 201623 Thomson Scientific, London, GB; AN 2015-77353R XP002767723, & CN 105 040 280 A (NANJING GEN HOSPITAL CHINESE PLA NANJING) 11 November 2015 (2015-11-11)

## Description

### TECHNICAL FIELD

This invention discloses composite meshes with anti-adhesive, antibacterial and hemostatic properties used after intraperitoneal hernia surgery to accelerate tissue regeneration and prevent post-surgery complications. Electro-spinning methods are applied to design mesh composites.

### BACKGROUND ART

Membranes, acting as a physical barrier and preventing adhesion of tissues are called "Anti-adhesive membranes". Adhesions of tissues are frequently observed after intraperitoneal and pelvic operations. Trauma, infection, pain and function disorder are other major problems for patients during healing process after such operations *[Linsky et al, 1991, Matsuzaki et al., 2014].* Especially after hernia surgery many of these complications are common and require additional medical treatment or even second surgery to remove mesh implant *[Lim et al., 2008, Collage et al., 2010].* Therefore anti-adhesive membranes are employed during operations in order to prevent or diminish dangerous complications *[Oesser et al., 2013, Obayan et al., 2013].* Biodegradable materials are generally preferred because they are removed from the body without medical intervention *[Oesser et al., 2013].* Polysaccharides, proteins, oxidized regenerated cellulose, sodium carboxymethyl cellulose, dextran sulfate, hyaluronic acid, chondroitin sulfate, polyglycolic acid and polylactic acid can be used alone or in combinations *[Lee et al., 2006].* However membranes from non-biodegradable materials are also common in clinical applications. These materials preventing adhesion may be designed in many different forms *[Stopek et al., 2013],* such as solution, gel or film*[Kim et al., 2015].* Anti-adhesive membranes in solid form are preferable to gel, foam or liquid formulations because of practical fixing of material *[Lee et al., 2006].*

The basis of modern hernia surgery begins with the use of synthetic patch (polyamide patch) by Usher (Polyamide patch) in 1958. Afterwards, braided polyester patch, polypropylene mesh, expandable polytetrafluoroethylene (PTFE) patches were used and these products have been their placed in the historical process in the repair of abdominal wall hernias *[Usher et al., 1963].* From the 1900's until today, varying patching materials have been introduced. In 1962, recurrence rate of 30-50% in the incisional hernia has started to decrease gradually with use of the monofilament polypropylene patch.

Repair with patch is the most widely used method in the treatment of hernia due to both reduction patient discomfort after surgery and the possibility of recurrence of the hernia *[Rodgers et al., 2000,Arroya et al., 2001].*

Synthetic materials can be used safely first in 1959 *[Klosterhalfen et al., 2005]* and developing of different materials have been released until today. There is no consensus ideas between researchers and clinicians about which material is ideal patch. Removal of the PTFE mesh from related region of the body in the case of infection inevitable. Incisional hernia recurrence is observed in 50% after primary repair. Incisional hernia repair with mesh in which a standard treatment is now recognized by all surgeons*[Leber et al., 1998].*

Composite meshes are widely used in the treatment of hernia. When the difficulties encountered in the use of existing composite meshes consider, the main problems faced in almost all outdoor operations are occurrence of infections, bleeding, biomaterials and tissue incompatibility. In this area, different structures and features developed by polymers and biomaterials market defines 'composite patch' which has given promising results in tissue-biomaterial compatibility, however, ideal mesh with the desired characteristics have not been developed yet. Composite meshes are used in both intraperitoneal and extraperitonal operations. The composite meshes used in intraperitoneal operations are usually bilayer and it is originated a non-biodegradable layer such as PP and the other layer consists of minimizing visceral adhesion risk. Because visceral adhesion occurs in the first 1-2 weeks of the postoperative period, the other side may comprise biodegradable polymers as well as permanent ones. The composite meshes used in extraperitoneal operations have generally a homogeneous composite structure to indicate the same properties throughout the material on abdominal wall.

Electrospun method is an appropriate method to obtain high surface/volume ratio *[Lee et al., 2006].* In this method, nanofibers with desired thickness and pore size can be pratically obtained by changing the voltage, solution concentration, flow rate, the distance between needle tip and collector. In addition, porosity is an important parameter for tissue growth and regeneration therefore, it makes this method advantageous.

In the U.S. Patent No. 5,002,551*,* oxidized regenerated cellulose fabric is used for prevention of intraperitoneal adhesions after surgery and then, to determine the impact, rabbits were sacrificed at the end of the 2 weeks of the *in vivo* study and it was reported that adhesion was decreased. In the EU. Patent No. 0 262 890 A2*,* oxidized regenerated cellulose containing heparin is designed to be used as adhesion preventive membrane. This membrane provided a physical barrier properties between area of surgical activity and neighboring tissue, also indicated that it is not toxic and has anti-adhesion effect.

In the U.S. Patent No. 5,364,622*,* hydroxyethyl cellulose hydrogel which comprises tissue plasminogen activator (tPA) is used to prevent adhesion of organs which may occur after the operation and it stated to possess antiadhesive effect.

The U.S. Patent No. 6,630,167 B2 describes foam of foam solution containing non crosslinked hyaluronic acid, crosslinked hyaluronic acid and aqueous solution mixture of both. Antiadhesive membranes was obtained from these solutions.

In the *Patent No.* 6,150,581 describes a method in which defect area in dog abdominal wall is coated with the alginate solution by spraying method and after that the surface region coated with alginate is again coated with chitosan solution to get rid of adhesion in application area.

In the U.S. Patent No. 5,795,584*,* films which generated from synthesized biodegradable copolymer is placed on polypropylene (PP) mesh by impression with PTFE coated plate and thus it is obtained as a multilayer barrier. The polymers used in this study are glycolide/trimethylene carbonate, glycolide/lactide, glycolide/lactide/trimethylene carbonate copolymers.

The U.S. Patent No. 2001/0008930 A1 *and* U.S. Patent No. 5,614,587 *studies,* collagen which is a protein having biocompatible, non-toxic and biodegradable feature is used as post operative adhesion prevention material.

In the WO Patent No. 2007/029913 A1*,* multilayer membranes which have hydrophilic and hydrophobic layers containing nano size fibers and antiadhesive property is designed by electrospun method. The hydrophobic layer consists of at least one of peptide, amino acid, polysaccharide, polyorthoester, polycarbonate, polyamide esters, polyalpha-cyanoacrylate and polyphosphazene.

The U.S. Patent No. 2002/0173213 A1 discloses biodegradable and/or absorbable materials obtained as fiber from polymer via electrospun method in order to reduce post-operative adhesion. This study uses glycolide, lactide, dioxane, caprolactone, trimethylene carbonate and comprises ethylene glycol and lysine as bioabsorbable monomers and also these monomers can be used to be homopolymer or copolymer.

The CN Patent No. 102908677 A describes a hernia patch containing polycaprolactone (PCL) on a polylactic-co-glycolic acid (PLGA) layer obtained by electrospun method to diminish adhesion in applied area. WO 2008/075398 A2 involves coating by electrospinning method on polypropylene mesh. This mesh is coated on one or two surfaces with a film of polymer material that will prevent the formation of tackiness and/or reduce the occurrence of erosion. According to another embodiment, the mesh is coated with a polymer fiber web. The coating network of polymer fibers and nano fibers was performed by electrospinning technique. Although many polyesters and polysaccharides have been proposed for this method, the neutral solution has not been mentioned in an effort to neutralize chitosan, which is an important parameter, especially by antibacterial effect, and has not been suggested in a non-acidic solvent.

### TECHNICAL PROBLEM

Hydrogels and solid films used as adhesion barriers have been introduced on the market for more than 30 years. On the other hand, combination of a standard mesh and material with anti adhesion property is a much more new area. Biodegradable or non-biodegradable meshes which can be single or multiple layer are used to prevent adhesion. However, these materials have not antibacterial and/or hemostatic properties to prevent post-operative complication.

### TECHNICAL SOLUTION

In order to solve the aforementioned problems, this study is aimed to prevent post-operative complications such as infection, adhesion formation and bleeding by means of newly develop multifunctional biomaterials which has anti adhesive, antibacterial and hemostatic properties.

### ADVANTAGEOUS EFFECTS

Developed products can be applied in the industrial field besides their antibacterial, hemostasis and anti adhesive properties. Thus, resulting products which have these properties add significance to the invention.

### BEST MODE

The best mode of the invention is designed composite layer which is PLGA blend system containing by weight of 10% and 30% chitosan on PP mesh. However, the mentioned examples should not be limited.

### INDUSTRIAL APPLICABILITY

The anti-adhesion barrier feature of present invention is useful regarding industrial applicability. The multi layered meshes which has non-toxic, antibacterial, hemostatic and tissue regeneration properties can solve the problems of post operative complications and thus gives the best convenience results when applied to operation areas.

### SUMMARY OF THE INVENTION

The scope of the invention is defined by the appended claims. The more generic description of the invention is provided for illustrative purposes only. A composite mesh which can be used for intraperitoneal hernia surgery was developed. The mesh accelerates tissue regeneration and has anti-adhesive, antibacterial, hemostatic properties. The product is described below;
- For intraperitoneal applications;bilayer membranes were obtained with coated polymeric blend systems prepared with polyester-based polymers and chitosan by electrospun method on the PP mesh woven material that has specific pore spacing.

### DESCRIPTION OF FIGURES

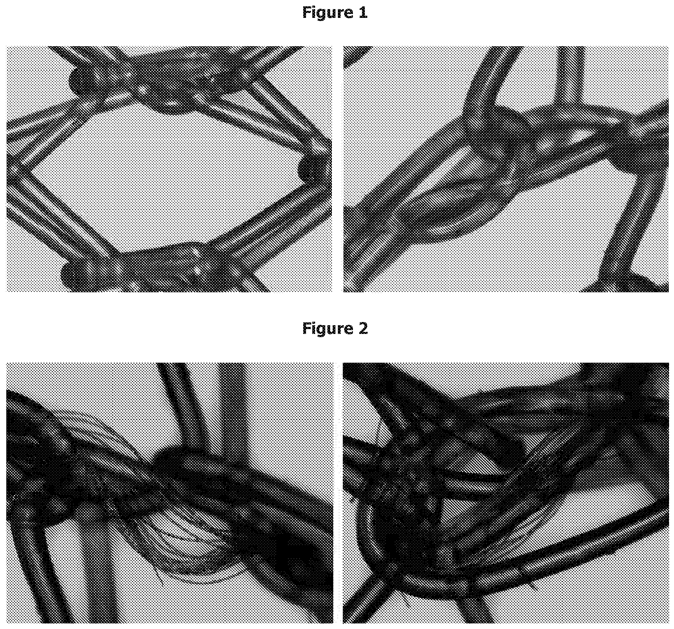

Figure 1. Microscope image of PP mesh obtained from the monofilament yarn
Figure 2. Microscope image of composites meshes acquired by weave of multi-filaments polyester, polypropylene based yarns

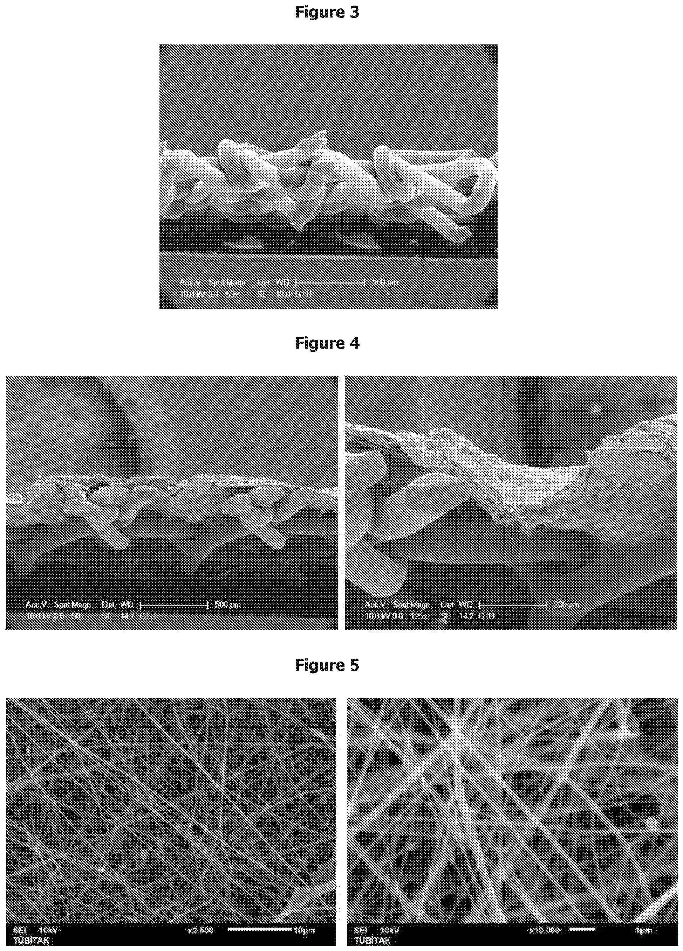

Figure 3. Cross section SEM images of monofilament PP mesh
Figure 4. Cross section SEM images of monofilament PP mesh coated with polysaccharides by electrospun method
Figure 5. Surface SEM images of PP mesh coated with polysaccharides by electrospun method

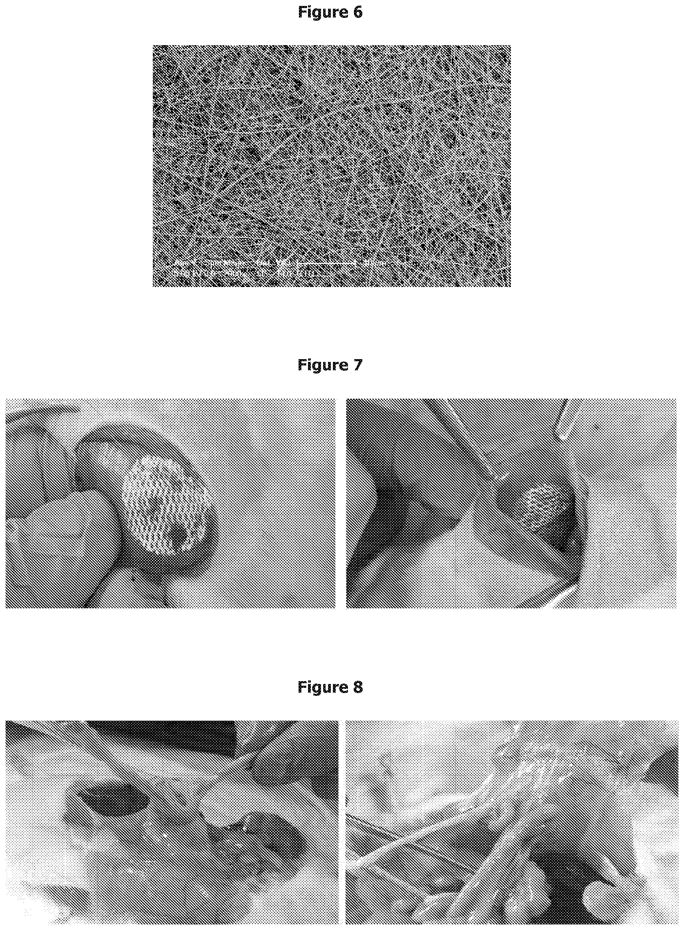

Figure 6. Surface SEM images of PP Mesh coated with PLGA/Chitosan%50 by electrospun method
Figure 7. Implant figures of PP mesh coated with polyester/polysaccharide blend system by electrospun method
Figure 8. Photographs of *in vivo* studies containing PP mesh implant

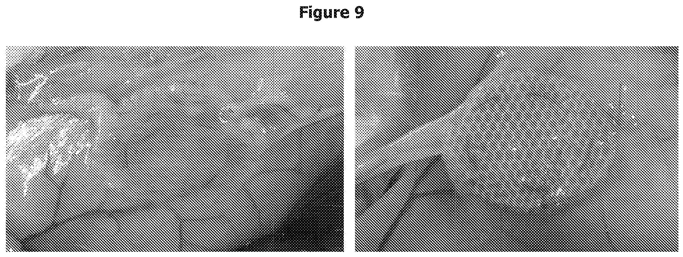

Figure 9. Photographs of *in vivo* studies of PP mesh coated with PLGA/Chitosan%30

### DETAILED DESCRIPTION OF THE INVENTION

In the present invention, biocompatible, antibacterial, hemostatic and anti-adhesive meshes for intraperitoneal applications were developed. Details of production steps and resulting products are indicated below.

### Process Steps:

### Step 1. Preparation of solutions and Blend System

- 4% weight solution of chitosan and PLGA was prepared in HFIP solvent and stirred with a magnetic stirrer for 12 h at ambient conditions. Polysaccharide ratio is about 30% of the total material in prepared blend system. The filtration was performed to remove the dissolved chitosan particles.
- 6% weight solution of chitosan and PLGA was prepared in HFIP solvent, stirring with a magnetic stirrer for 12 h at ambient conditions. Polysaccharide ratio is about 10% of the total material in prepared blend system. The filtration was performed to remove the dissolved chitosan particles.

### Step 2. Application of Electrospun Blend System

Solution and blend systems were put into syringe and it was placed in a pump of electrospun device. PP mesh was coated on the aluminum cylinder then; optimized parameter values of voltage, the distance between needle tip and collector and flow rate were applied. Fiber structure was homogeneously collected on the PP mesh with the rotation of the aluminum cylinder. After the desired amount of product collected, bilayer mesh is completely dried at 40°C vacuum oven for 1 day.

### REFERENCES

1. Linsky C.B., (1991). "Method and Material for Prevention of Surgical Adhesion". U.S. Patent 5,002,551.
2. Matsuzaki M. (2014). "Anti-Adhesion Membrane". E.P 2 740 496 A1.
3. Lim, R.,Morrill, J. M., Lynch, R. C., Reed, K. L., Gower, A. C., Leeman, S. E., Stucchi, A. F., Becker, J. M. Journal of Gastrointestinal Surgery. (2008). 13, 35-42.
4. Collage, R. D.,Rosengart, M. R. Surgical Infections. (2010). 11, 311-318.
5. Oesser S., (2013). "Use of a Medical Implant as Adhesion Barrier". U.S. Patent 2013/0317611 A1.
6. Obayan A.O.E., (2013). "Reducing Post Operative Adhesion Formation with Intraperitoneal Glutamine". U.S. Patent 2013/0171209 A1.
7. Lee Y.W., (2006). "Multi-Layered Antiadhesion Barrier". WO 2007/029913 A1.
8. Stopek J., (2013). "Implantable Devices Including a Mesh and a Pivotable Film". U.S. Patent 2013/0030360 A1.
9. Kim S.W., (2015). "Composition for Anti-Adhesion, Surgical Mesh Composite with Anti-Adhesion Property Comprising the Same and Method for ProducingThereof". U.S. Patent 2015/0045818 A1.
10. Usher FC. Hernia repair with knitted polypropylene mesh. Surg Gynecol Obstet; 117:239-240, 1963.
11. Rodgers, A.,Walker, N., Schug, S.,McKee, A.,Kehlet, H.,van Zundert, A., Sage, D.,Futter, M., Saville, G., Clark, T., S. Mac. Mahon., S. BMJ. (2000). 321, 1-12.
12. Arroyo, A.,Garcia, P., Perez, P., Andreu, J., Candela, F., Calpena. R. British Journal of Surgery. (2001). 88, 1321-1323.
13. Klosterhalfen, B.,Junge, K., Klinge, U. Expert Review of Medical Devices. (2005). 2, 103-117.
14. Leber, G. E.,Garb, J. L., Alexander, A.I., Reed, W. P. Arch. Surg. (1998). 133, 378-382.
15. Linsky C.B., (1987). "Heparin-Containing Adhesion Prevention Barrier and Process". EU. Patent 0 262 890 A2.
16. Franz H., (1994). "Methods for Preventing Adhesion to Organs and Parts of Organs by Application of Tissue Plasminogen Activator and Hydroxyethylcellulose Hydrogel". U.S. Patent 5,364,622.
17. Zhang G., (2003). "Hyaluronic Acid Anti-AdhesionBarrier". U.S. Patent 6,630,167 B2.
18. Jiang Y., (2000). "Chitosan-Alginate Anti-Adhesion Barrier". U.S. Patent 6,150,581.
19. Totakura N., (1998). "Post-Surgical Anti-Adhesion Device". U.S. Patent 5,795,584.
20. Tayot J.L., (2001). "Collagenic Material Useful in Particular for Preventing Post-Operative Adhesions". U.S. Patent 2001/0008930 A1.
21. Rhee W.M., (1997). "Collagen- Based Bioadhesive Composition". U.S. Patent 5,614,587.
22. Chu B., (2002). "Biodegradable and/or Bioabsorbable Fibrous Articles and Methods for Using the Articles for Medical Application". U.S. Patent 2002/0173213 A1.
23. Zhichao H., (2013). "Preparation Method of Anti-Adhesion Absorbable Hernia Patch". CN 102908677 A.

## Claims

1. Composite mesh having biocompatible, antibacterial, hemostatic and anti-adhesive properties comprising a non-degradable mesh; the said mesh is prepared with blend system of biodegradable polyester-based polymers/ chitosan mixture in hexafluoro isopropanol (HFIP) solvent and coated on polypropylene layer as nanofiber via electrospun method.

2. The mesh of claim 1, wherein polyester used is polyglycolic acid, polylactic acid, polyglycolic-co-lactic acid, polytrimethylenecarbonate, polyglycolic-co-trimethylene carbonate, polylactic-co-trimethylene carbonate, polycaprolactone, polyglycolic-co-caprolactone, polylactic-co-caprolactone, politrimetilencarbonate-co-caprolactone.

3. The mesh of claim 1, wherein the molecular weight of polyester (Mₙ) is from 10.000 to 1.000.000 Da.

4. The mesh claim 1, wherein weight ratio of polyester/chitosan mixture to HFIP in prepared blend system is between 1-90%.

5. The mesh of claim 1, wherein the weight ratio of chitosan to polyester in prepared blend system is between 0.1 to 99.1%.

6. The mesh of claim 1, wherein polyester used is preferably polyglycolic-co-lactic acid (PLGA).

7. The mesh of claim 1, wherein solution amount of chitosan/polyester mixture in prepared blend system is 4% by weight.

8. The mesh of claim 7, wherein chitosan weight ratio in chitosan/polyester mixture is 30% for prepared blend system with HFIP.

9. The mesh of claim 1, wherein solution amount of chitosan/polyester mixture in prepared blend system is 6% by weight.

10. The mesh of claim 9, wherein chitosan weight ratio in chitosan/polyester mixture is 10% for prepared blend system with HFIP.

11. The mesh of claim 1, wherein polyester filament number for polypropylene/polyester woven material is from 1 to 100.

12. The mesh of claim 1, wherein porosity of polyester/chitosan layer is between 10-80% and pore size of polyester/chitosan layer is from 10 nm to 10 microns.

13. The mesh of claim 1, wherein nanofiber diameter of polyester/chitosan produced by electrospinning is from 10 to 1000 nm.

## Patentansprüche

1. Zusammengesetztes Netz mit biokompatiblen, antibakteriellen, hämostatischen und anti-adhäsiven Eigenschaften, umfassend ein nicht-abbaubares Netz; das genannte Netz mit einem Mischungssystem aus biologisch abbaubarem Polymer/Chitosan-Gemisch auf Polyesterbasis in Hexafluorisopropanol (HFIP)-Lösungsmittel zubereitet und als Nanofaser mittels Elektrospinnverfahren auf eine Polypropylenschicht beschichtet ist.

2. Netz nach Anspruch 1, wobei der verwendete Polyester; Polyglycolsäure, Polymilchsäure, Polyglycol-Ko-Milchsäure, Polytrimethylencarbonat, Polyglycol-Ko-Trimethylencarbonat, Polymilch-Ko-Trimethylencarbonat, Polycaprolacton, Polyglycol-Ko-Caprolacton, Polymilch-Ko-Caprolacton, Politrimetilencarbonat-Ko-Caprolacton ist.

3. Netz nach Anspruch 1, wobei das Molekulargewicht des Polyesters (Mₙ) von 10.000 bis 1.000.000 Da beträgt.

4. Netz nach Anspruch 1, wobei das Gewichtsverhältnis von Polyester/Chitosan-Gemisch zu HFIP im vorbereiteten Mischungssystem zwischen 1-90% liegt.

5. Netz nach Anspruch 1, wobei das Gewichtsverhältnis von Chitosan zu Polyester in einem vorbereiteten Mischungssystem zwischen 0.1 bis 99.1% liegt.

6. Netz nach Anspruch 1, wobei der verwendete Polyester vorzugsweise Polyglykol-Ko-Milchsäure (PLGA) ist.

7. Netz nach Anspruch 1, wobei die Lösungsmenge der Chitosan/Polyester-Gemisch im vorbereiteten Mischungssystem 4 Gew.-% liegt.

8. Netz nach Anspruch 7, wobei das Chitosan-Gewichtsverhältnis in der Chitosan/Polyester-Gemisch 30% liegt für ein vorbereitetes Mischungssystem mit HFIP.

9. Netz nach Anspruch 1, wobei die Lösungsmenge der Chitosan/Polyester-Gemisch im vorbereiteten Mischungssystem 6 Gew.-% liegt.

10. Netz nach Anspruch 9, wobei das Chitosan-Gewichtsverhältnis in der Chitosan/Polyester-Gemisch 10% liegt, für ein vorbereitetes Mischungssystem mit HFIP.

11. Netz nach Anspruch 1, wobei die Polyester-Filamentzahl für gewebtes Polypropylen/Polyester-Material von 1 bis 100 beträgt.

12. Netz nach Anspruch 1, wobei die Porosität der Polyester/Chitosan-Schicht zwischen 10-80% liegt und die Porengröße der Polyester/Chitosan-Schicht von 10 nm bis 10 Mikron beträgt.

13. Netz nach Anspruch 1, wobei der Nanofaserdurchmesser von Polyester/Chitosan, das durch Elektrospinnen hergestellt wird, von 10 bis 1000 nm beträgt.

## Revendications

1. Maille composite ayant des propriétés biocompatibles, antibactériennes, hémostatiques et antiadhésives comprenant une maille non dégradable ; ladite maille est préparée avec un système de mélange de polymères biodégradables basés sur polyester/de chitosane dans le solvant hexafluoro isopropanol (HFIP) et enduite sur une couche de polypropylène en tant que nanofibre par la méthode d'électrofilage.

2. Maille selon la revendication 1, dans laquelle le polyester utilisé est l'acide polyglycolique, l'acide polylactique, l'acide polyglycolique-co-lactique, le polytriméthylènecarbonate, le polyglycolique-co-triméthylène carbonate, le polylactique-co-triméthylène carbonate, le polycaprolactone, le polyglycolique-co-caprolactone, le polylactique-co-caprolactone, le polytriméthylènecarbonate-co-caprolactone.

3. Maille selon la revendication 1, dans laquelle le poids moléculaire du polyester (Mₙ) est de 10.000 à 1.000.000 Da.

4. Maille selon la revendication 1, dans laquelle le rapport pondéral du mélange polyester/chitosane au HFIP dans le système de mélange préparé est compris entre 1-90%.

5. Maille selon la revendication 1, dans laquelle le rapport pondéral du chitosane au polyester dans le système de mélange préparé est compris entre 0.1 à 99.1%.

6. Maille selon la revendication 1, dans laquelle le polyester utilisé est de préférence l'acide polyglycolique-co-lactique (PLGA).

7. Maille selon la revendication 1, dans laquelle la quantité de solution du mélange chitosane/polyester dans le système de mélange préparé est de 4% en poids.

8. Maille selon la revendication 7, dans laquelle le rapport pondéral du chitosane dans le mélange chitosane/polyester est de 30% pour le système de mélange préparé avec le HFIP.

9. Maille selon la revendication 1, dans laquelle la quantité de solution du mélange chitosane/polyester dans le système de mélange préparé est de 6% en poids.

10. Maille selon la revendication 9, dans laquelle le rapport pondéral du chitosane dans le mélange chitosane/polyester est de 10% pour le système de mélange préparé avec le HFIP.

11. Maille selon la revendication 1, dans laquelle le numéro de filament de polyester pour la matière tissée en polypropylène/polyester est de 1 à 100.

12. Maille selon la revendication 1, dans laquelle la porosité de la couche de polyester/chitosane est comprise entre 10-80% et la taille des pores de la couche de polyester/chitosane est de 10 nm à 10 microns.

13. Maille selon la revendication 1, dans laquelle le diamètre des nanofibres de polyester/chitosane produites par électrofilage est de 10 à 1000 nm.
